# FASCICULE DE BREVET EUROPEEN

(11) **EP 2 056 105 B1**
(45) Date de publication et mention de la délivrance du brevet: **22.09.2010**
(21) Numéro de dépôt: 08354078.1
(22) Date de dépôt: 22.10.2008
(51) Int. Cl.: G01N 33/24, E02D 1/02

(54) **Dispositif de mesure de l'érosion du sol à un niveau inférieur à la surface et procédé de mesure**
Vorrichtung zur Bodenerosionsmessung unterhalb der Bodenoberfläche und Messverfahren
Device for measuring soil erosion at a level below the surface and measurement method

(30) Priorité: 31.10.2007 FR 0707668
(43) Date de publication de la demande: 06.05.2009
(73) Titulaire: IMS RN - Ingénierie des Mouvements de Sol et des Risques Naturels, 38330 Montbonnot Saint Martin (FR)
(72) Inventeur: Plotto, Pierre, 38700 Corenc (FR)
(74) Mandataire: Hecké, Gérard

(56) Documents cités:
- GB-A- 2 221 762
- US-A- 3 690 166
- US-A- 5 331 847
- US-A- 6 021 664

## Description

### Domaine technique de l'invention

L'invention concerne un dispositif de mesure de l'érosion du sol à un niveau inférieur à la surface.

### État de la technique

Il existe des dispositifs de mesure permettant de mesurer l'érosion d'un sol. Le document US 5,331,847 dont le dispositif est illustré schématiquement à la figure 1, divulgue un tel dispositif comportant une réserve 1 d'eau stockée dans un réservoir extérieur, l'eau contenue dans ce réservoir étant ensuite envoyée sous pression dans un tube creux 2, le tube creux 2 comportant un trou d'injection 8 dirigé vers le sol 3 dont on l'érosion est à mesurer. Avant de réaliser une mesure, le sol 3 est mis à plat, il est ensuite saturé en eau pour éviter que l'eau injectée ne soit absorbée par le sol 3. Une mesure du profil initial 4 du sol 3 est effectuée pour servir de base. De l'eau sous pression est ensuite éjectée, sous forme de jet, contre le sol par le trou d'injection 8 et provoque, en fonction de la vitesse de cette dernière, l'érosion du sol 3 au niveau du point d'impact du jet d'eau. Une seconde mesure du profil du sol 3 est effectuée puis le volume du sol 3 enlevé par cette eau sous pression est ensuite déterminé en se basant sur les différences entre le profil initial 4 et le profil modifié 5 par l'érosion. Si un tel dispositif permet effectivement de mesurer l'érosion du sol 3 en surface, il ne permet en aucun cas de mesurer l'érosion d'un sol en profondeur. C'est-à-dire à un niveau inférieur à la surface.

Le document GB2221762 décrit un dispositif pour déterminer l'érosion d'un sol. Le dispositif comporte des moyens d'injection pour produire un flux d'eau au niveau du sol. Le flux forme alors un trou dans le sol par érosion. L'érosion est déterminée par la profondeur du trou obtenue pour un flux constant en fonction du temps. Un tel dispositif ne permet donc pas de mesurer l'érosion d'un sol en profondeur.

### Objet de l'invention

L'objet de l'invention consiste à réaliser un dispositif permettant de mesurer de l'érosion en profondeur, à un niveau inférieur à la surface.

Ce but est atteint par le fait que le dispositif comporte :
- des moyens d'injection d'un liquide, reliés à une première extrémité d'un premier tube creux d'injection enfoncé dans le sol, ledit premier tube creux comportant à une seconde extrémité une tête au-dessus de laquelle est réalisé au moins un trou d'injection du liquide dans le sol, lesdits moyens d'injection comportant des moyens de contrôle et de mesure du débit d'injection,
- des moyens de récupération du liquide depuis le sol audit niveau inférieur, comportant d'une part au moins un trou de captage du liquide, communiquant avec une chambre, et d'autre part des moyens de transfert, vers un turbidimètre prévu à la surface, du liquide récupéré dans la chambre, les moyens de transfert comportant un second tube creux d'expulsion, d'axe sensiblement parallèle au premier tube creux d'injection.

Selon une variante, le premier tube creux d'injection et le second tube creux d'expulsion sont coaxiaux, le second tube creux d'expulsion étant interne au tube et le trou d'injection du liquide, la chambre se situant dans la tête. Un premier fluxmètre est disposé entre le trou d'injection et le trou de captage pour mesurer la vitesse de transit du liquide dans le sol le long du premier tube creux.

Selon un mode de réalisation préférentiel, les moyens de transfert du liquide comportent une pompe de refoulement intégrée dans la tête, ayant une entrée reliée à la chambre et une sortie reliée au second tube creux d'expulsion.

Selon un autre mode de réalisation préférentiel, les moyens de transfert de liquide comportent un compresseur à air relié à un tuyau interne et coaxial au second tube creux d'expulsion pour récupérer le liquide par remontée d'air.

L'invention concerne aussi un procédé de mise en oeuvre du dispositif, **caractérisé en ce que :**
- on enfonce le premier tube creux d'injection et les moyens de récupération dans le sol jusqu'à un niveau inférieur à la surface,
- on injecte périodiquement le liquide, selon un débit donné, dans le premier tube creux d'injection grâce aux moyens d'injection, en augmentant le débit selon une valeur d'incrémentation prédéterminée entre deux injections,
- on effectue, pour chaque injection, une mesure de la turbidité de l'eau récupérée par les moyens de récupération,
- on établit une courbe de mesure associant le débit aux valeurs mesurées de turbidité,
- on contrôle la valeur de la pente de la courbe de mesure, et on détermine un point de rupture correspondant à une augmentation brutale de ladite valeur de pente,
- on détermine le débit correspondant au point de rupture.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :
La figure 1 illustre un dispositif de mesure de l'érosion selon l'art antérieur.
Les figures 2 à 6 illustrent chacune un mode de réalisation de dispositif selon l'invention.

### Description de modes particuliers de réalisation

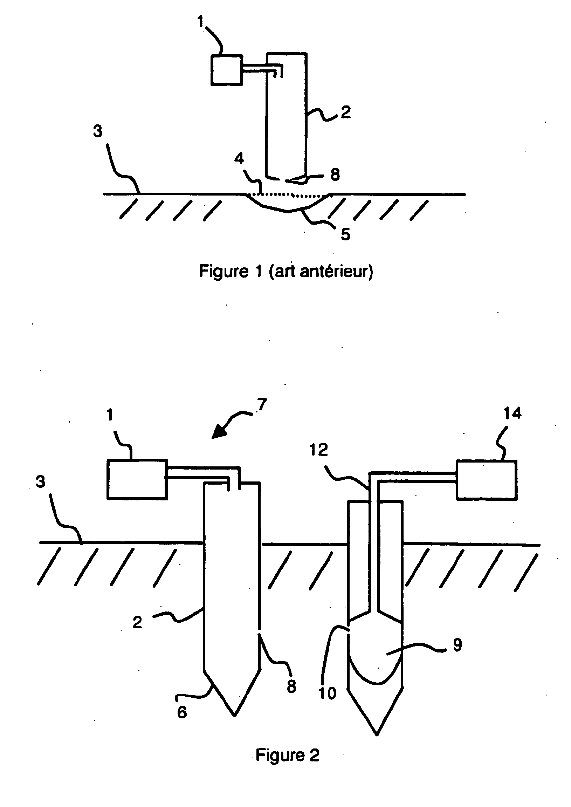

Selon un premier mode de réalisation illustré à la figure 2, un dispositif de mesure de l'érosion du sol à un niveau inférieur à la surface se présente en deux parties. La première partie est constituée par un premier tube creux 2 d'injection comportant à une extrémité une tête 6 et à l'autre extrémité des moyens d'injection 7 d'un liquide. Pendant son utilisation pour la mesure de l'érosion, le tube creux 2 est enfoncé dans le sol 3 (voir figure 2). Les moyens d'injection 7 sont reliés, d'une part, hermétiquement à l'extrémité correspondante du premier tube creux 2, par exemple par des joints d'étanchéités, et d'autre part à une réserve de liquide 1. Les moyens d'injection comportent des moyens de contrôle et de mesure du débit. Le premier tube creux 2 comporte au moins un trou d'injection 8 situé au-dessus de la tête 6, ce trou permettant d'injecter dans le sol 3 le liquide contenu dans le premier tube creux 2. La seconde partie du dispositif correspond à des moyens de récupération du liquide depuis le sol. Ces moyens de récupération comportent d'une part au moins un trou de captage 10 du liquide communiquant avec une chambre 9 et d'autre part des moyens de transfert comportant un second tube creux 12 d'expulsion reliant la chambre 9 à un turbidimètre prévu à la surface. La chambre 9 est aménagée à la partie inférieure du second tube creux 12. Le trou de captage 10 est pratiqué dans la paroi du second tube 12 au niveau de la chambre 9. Les première et seconde parties du dispositif seront enfoncées de manière sensiblement parallèle dans le sol, la tête 6 et la chambre 9 se retrouvant chacune à un niveau inférieur à la surface, ces niveaux étant de préférence les mêmes. Dans ce premier mode de réalisation, le second tube 12 est indépendant du premier tube 2 et disposé à l'extérieur et à proximité de ce dernier, dans une configuration où le trou d'injection 8 est sensiblement en regard du trou de captage 10.

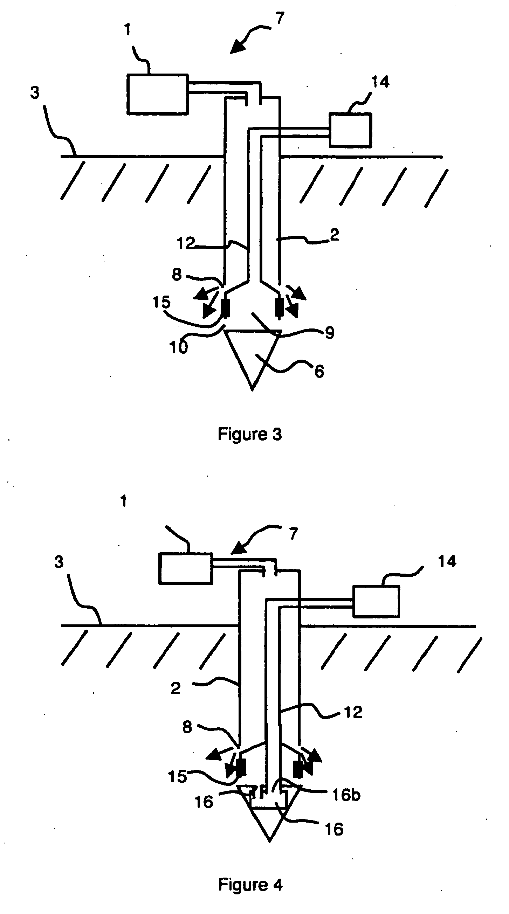

Selon un deuxième mode de réalisation illustré à la figure 3, les premier et second tubes creux sont coaxiaux, le second tube creux 12 étant interne au premier tube creux 2. Le trou de captage 10 est intercalé entre la tête 6 du premier tube creux 2 et le trou d'injection 8 du liquide, la chambre 9 se situe dans la tête 6 du premier tube creux 2. De plus le dispositif comporte un fluxmètre 15 destiné à mesurer la vitesse de transit, aussi appelée vitesse de percolation, du liquide dans le sol 3 le long du premier tube creux 2. Ce fluxmètre 15 est disposé contre le premier tube creux 2 entre le trou d'injection 8 et le trou de captage 10. A titre d'exemple, le fluxmètre 15 pourra être de type thermorésistant ou à ultrasons, ou à polarisation électrique spontanée ou autre.

L'utilisation de l'un quelconque des deux dispositifs de mesure décrits ci-dessus consiste à augmenter, de manière périodique, le débit du liquide injecté dans le tube creux 2 afin d'obtenir une courbe de mesure de la turbidité mesurée en fonction du débit d'injection, qui sera ensuite contrôlée pour déterminer le seuil critique d'érosion interne. Selon un mode préférentiel, après que le dispositif soit enfoncé dans le sol 3 au niveau (inférieur à la surface) où l'on veut effectuer la mesure de l'érosion, chaque mesure de turbidité est réalisée par le cycle formé par les cinq étapes suivantes :
- le liquide est Injecté, selon un débit donné dans le premier tube creux 2 grâce aux moyens d'injection 7, le débit d'injection est contrôlé par les moyens de contrôle du débit, ce qui crée une certaine pression dans le premier tube creux 2 gardée constante tant que la mesure n'est pas réalisée,
- le liquide, sous pression constante, est automatiquement injecté dans le sol par l'intermédiaire du trou d'injection 8,
- une partie de ce liquide injecté peut être absorbée par le sol du fait de son coefficient de perméabilité, l'autre partie s'écoulant dans le sol est ensuite au moins partiellement récupérée dans la chambre 9, par l'intermédiaire du trou de captage 10. Ce liquide est alors chargé par des particules arrachées au sol dont il s'est chargé lors de son transit dans le sol entre le trou d'injection 8 et le trou de captage 10. La chambre 9 a de préférence une base incurvée ce qui permet de retenir le liquide s'écoulant à l'intérieur,
- le liquide présent dans la chambre 9 est expulsé jusqu'au turbidimètre 14 grâce aux moyens de transfert,
- on effectue une mesure de la turbidité du liquide récolté, et l'on associe cette valeur mesurée au débit.

Chaque mesure de turbidité, associée à un débit correspondant selon la dernière étape du cycle, est réalisée par un cycle unitaire constitué par les cinq étapes ci-dessus. La courbe de mesure est obtenue en réalisant périodiquement de telles mesures de turbidité pour lesquelles le débit à été successivement augmenté dans la première étape du cycle. Autrement dit, on augmente le débit selon une valeur d'incrémentation prédéterminée entre deux injections. Le débit du liquide injecté dans le premier tube creux 2 est incrémenté grâce aux moyens de contrôle du débit, ce qui permet d'augmenter la pression dans le premier tube creux 2 et par voie de conséquence augmenter la vitesse d'injection du liquide dans le sol au niveau du trou d'injection 8. Après plusieurs mesures successives de la turbidité et de son débit associé, on obtient la courbe de mesure représentant la turbidité de l'eau récoltée en fonction du débit à l'injection. On contrôle, en temps réel ou en différé, la valeur de la pente de la courbe de mesure, dont la forme est une quasi-droite jusqu'à détecter une augmentation brutale de la valeur de pente. On détermine alors le débit correspondant au point de rupture de la pente. L'évolution de la pente de la courbe est dépendante du milieu et à titre d'exemple de paramètres essentiels tels que la granulométrie, la perméabilité ou encore la densité. Ce point correspond à un seuil critique de vitesse de transit du liquide dans le sol (à un niveau inférieur à la surface) entre le trou d'injection 8 et le trou de captage 10, et signifie que l'on se retrouve en situation d'érosion du sol. Dans la variante de réalisation intégrant les moyens de récupération dans le premier tube creux (figure 3), en plus de la mesure du débit, on mesure la vitesse de transit du liquide dans le sol (ou percolation) par le fluxmètre 15 au moins au point de rupture ou à chaque mesure de turbidité pour associer cette vitesse de transit à la mesure de turbidité lui correspondant. La vitesse de transit mesurée par le fluxmètre 15 au point de rupture correspondant au seuil critique de l'érosion du sol.

Selon un mode de réalisation particulier illustré à la figure 4, les moyens de transfert comportent une pompe de refoulement 16, cette pompe étant située dans la tête 6. La pompe est reliée en entrée 16a à la chambre 9 et en sortie 16b au second tube creux 12 d'expulsion. Ainsi le liquide contenu dans la chambre 9 est aisément expulsé vers le turbidimètre 14 par transfert dans le second tube creux 12. De plus, la pompe 16 permet de mesurer le débit de liquide récupéré et la dépression créée dans la chambre 9.

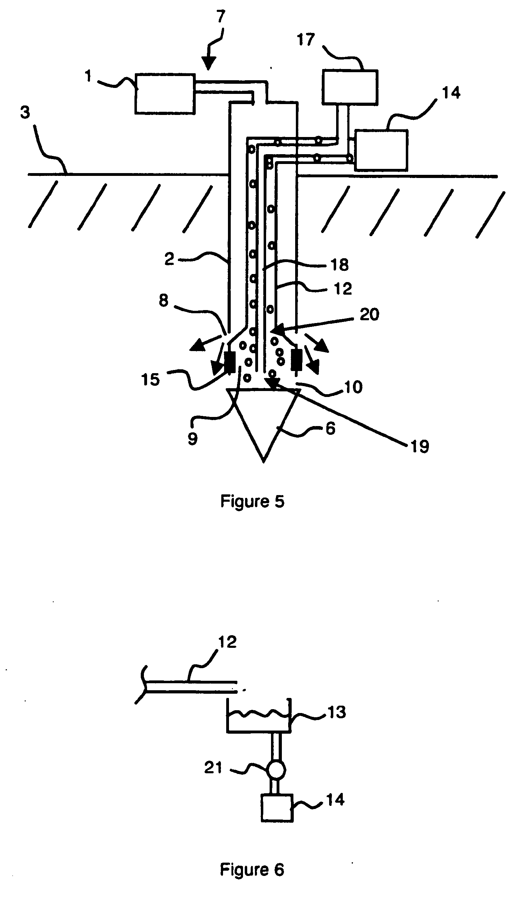

Dans un autre mode de réalisation illustré à la figure 5, les moyens de transfert utilisent un compresseur à air 17, relié à un tuyau 18 d'acheminement de l'air du compresseur. De préférence, le tuyau 18 est interne au second tube creux 12 de préférence coaxialement. Dans tous les cas, le tuyau 18 débouche dans la chambre 9 et de préférence descendant jusqu'à une partie inférieure 19 de la chambre 9 et le second tube creux débouchant sur une partie supérieure 20 de la chambre 9. La partie inférieure 19 de la chambre peut avoir une forme incurvée ce qui facilite la remontée du liquide par poussée d'air (« air-lift » en anglais). Le principe de la poussée d'air est le suivant, le compresseur 17 envoi de l'air dans le tuyau 18, ce qui crée au niveau de la chambre 9 une grande quantité de bulles. Ces bulles vont remonter dans le tuyau creux 12 d'expulsion. Les forces de tension existant à la surface des bulles vont obliger le liquide récupéré dans la chambre 9 à accompagner les bulles dans leur remontée, de préférence, pour augmenter ces forces de tension, le second tube creux 12 d'expulsion aura une petite section. Ce mécanisme est accentué par une surpression de l'air par rapport à la pression hydrostatique. Cette surpression ne doit cependant pas être trop forte pour éviter d'injecter de l'air dans le sol à travers le trou de captage 10. Dans une variante, le second tube creux 12 d'expulsion comporte un fluxmètre permettant de mesurer le débit de remontée du liquide. A titre d'exemple, le fluxmètre pourra être de type thermorésistant ou à ultrasons, ou à polarisation électrique spontanée ou autre.

Comme l'illustre la figure 6, et quel que soit le mode de réalisation précédent, le second tube creux 12 d'expulsion peut être relié à un réceptacle 13, lui-même relié au turbidimètre 14 par des moyens de remplissage du turbidimètre. Les moyens de remplissage correspondent à un circuit hydraulique comportant une vanne 21 qui peut être actionnable manuellement ou commandée par un circuit de traitement contrôlant les mesures pouvant être effectuées par le turbidimètre. Une fois acheminé dans le réceptacle 13, le liquide est donc transféré au turbidimètre 14 pour mesure de sa turbidité.

Dans une variante, chaque extrémité des tubes du dispositif devant être enfoncée dans le sol présente un profil en forme de pointe ce qui facilite l'enfoncement par battage ou l'enfoncement statique du dispositif au niveau inférieur au sol désiré.

De préférence, le liquide utilisé pour l'injection dans le sol est de l'eau.

Un tel dispositif permet de générer en profondeur dans le sol un flux de liquide, et de relever en sortie la valeur de la turbidité de ce liquide. La turbidité est proportionnelle à la quantité de matériau entraîné par le flux pour le débit injecté dans le premier tube creux. Lorsque la courbe, représentant la turbidité en fonction du débit d'injection, présente un point de rupture important, alors le seuil d'érosion du sol est considéré comme atteint.

## Revendications

1. Dispositif de mesure de l'érosion du sol à un niveau inférieur à la surface, **caractérisé en ce qu'**li comporte :
- des moyens d'injection (7) d'un liquide, reliés à une première extrémité d'un premier tube creux (2) d'injection enfoncé dans le sol, ledit premier tube creux comportant à une seconde extrémité une tête (6) au-dessus de laquelle est réalisé au moins un trou d'injection (8) du liquide dans le sol, lesdits moyens d'injection comportant des moyens de contrôle et de mesure du débit d'injection,
- des moyens de récupération du liquide depuis le sol audit niveau inférieur, comportant d'une part au moins un trou de captage (10) du liquide, communiquant avec une chambre (9), et d'autre part des moyens de transfert, vers un turbidimètre (14) prévu à la surface, du liquide récupéré dans la chambre, les moyens de transfert comportant un second tube creux (12) d'expulsion, d'axe sensiblement parallèle au premier tube creux (2) d'injection.

2. Dispositif selon la revendication 1, **caractérisé en ce que** le premier tube creux (2) d'injection et le second tube creux (12) d'expulsion sont coaxiaux, le second tube creux (12) d'expulsion étant interne au premier tube creux (2) d'injection, le trou de captage (10) étant intercalé entre la tête (6) du premier tube (2) et le trou d'injection (8) du liquide, la chambre (9) se situant dans la tête (6) et **en ce qu'**un premier fluxmètre (15) est disposé entre le trou d'injection (8) et le trou de captage (10) pour mesurer la vitesse de transit du liquide dans le sol le long du premier tube creux.

3. Dispositif selon les revendications 1 à 2, **caractérisé en ce qu'**il comporte un réceptacle (13) interposé entre le second tube creux (12) d'expulsion et des moyens de remplissage du turbidimètre (14).

4. Dispositif selon les revendications 1 à 3, **caractérisé en ce que** les moyens de transfert du liquide comportent une pompe de refoulement (16), ayant une entrée (16a) reliée à la chambre et une sortie (16b) reliée au second tube creux (12) d'expulsion.

5. Dispositif selon l'une quelconque des revendications 1 à 3, **caractérisé en ce que** les moyens de transfert du liquide comportent un compresseur à air (17) relié à un tuyau (18) interne et coaxial au second tube creux (12) d'expulsion et débouchant dans la chambre, pour récupérer le liquide par remontée d'air.

6. Dispositif selon la revendication 5, **caractérisé en ce que** le second tube creux (12) comporte un deuxième fluxmètre à thermorésistance.

7. Procédé de mise en oeuvre d'un dispositif selon l'une des revendications 1 à 6, **caractérisé en ce que:**
- on enfonce le premier tube creux (2) d'injection et les moyens de récupération dans le sol (3) jusqu'à un niveau inférieur à la surface,
- on Injecte périodiquement le liquide, selon un débit donné, dans le premier tube creux (2) d'injection grâce aux moyens d'injection (7), en augmentant le débit selon une valeur d'incrémentation prédéterminée entre deux injections,
- on effectue, pour chaque injection, une mesure de la turbidité du liquide récupéré par les moyens de récupération,
- on établit une courbe de mesure associant le débit aux valeurs mesurées de turbidité,
- on contrôle la valeur de la pente de la courbe de mesure, et on détermine un point de rupture correspondant à une augmentation brutale de ladite valeur de pente,
- on détermine le débit correspondant au point de rupture.

8. Procédé selon la revendication 7, **caractérisé en ce que** le dispositif comportant un premier fluxmètre (15) disposé entre le trou d'injection (8) et le trou de captage (10) pour mesurer la vitesse de transit du liquide dans le sol le long du premier tube creux, ladite vitesse est mesurée au moins au point de rupture.

## Claims

1. Device for measuring soil erosion at a level lower than the surface, **characterized in that** it comprises:
- injection means (7) of a liquid connected to a first end of a first hollow injection tube (2) sunk in the soil, said first hollow tube comprising, at a second end, a head (6) above which at least one injection hole (8) for injecting the liquid into the soil is made, said injection means comprising means for controlling and measuring the injection rate,
- means for recovering the liquid from the soil at said lower level, comprising on the one hand at least one collecting hole (10) of the liquid communicating with a chamber (9), and on the other hand means for transferring the liquid recovered in the chamber to a turbidimeter (14) provided at the surface, the means for transferring comprising a second hollow expulsion tube (12) of substantially parallel axis to the first hollow injection tube (2).

2. Device according to claim 1, **characterized in that** the first hollow injection tube (2) and the second hollow expulsion tube (12) are coaxial, the second hollow expulsion tube (12) being internal to the first hollow injection tube (2), the collecting hole (10) being fitted between the head (6) of the first tube (2) and the injection hole (8) of the liquid, the chamber (9) being situated in the head (6), and that a first flow meter (15) is arranged between the injection hole (8) and the collecting hole (10) to measure the speed of transit of the liquid in the soil along the first hollow tube.

3. Device according to claims 1 to 2, **characterized in that** it comprises a receptacle (13) fitted between the second hollow expulsion tube (12) and means for filling the turbidimeter (14).

4. Device according to claims 1 to 3, **characterized in that** the means for transferring the liquid comprise a discharge pump (16) having an inlet (16a) connected to the chamber and an outlet (16b) connected to the second hollow expulsion tube (12).

5. Device according to any one of claims 1 to 3, **characterized in that** the means for transferring the liquid comprise an air compressor (17) connected to a pipe (18), internal and coaxial to the second hollow expulsion tube (12) and opening out into the chamber, to recover the liquid by air upflow.

6. Device according to claim 5, **characterized in that** the second hollow tube (12) comprises a second thermoresistance flow meter.

7. Method for implementing a device according to one of claims 1 to 6, **characterized in that**:
- the first hollow injection tube (2) and the means for recovering are sunk into the soil (3) to a lower level than the surface,
- the liquid is injected into the first hollow injection tube (2) periodically, with a given flow rate, by means of the injection means (7), the flow rate being increased by a preset incrementation value between two injections,
- measurement of the turbidity of the liquid recovered by the means for recovering is made for each injection,
- a measurement curve is established associating the flow rate with the measured turbidity values,
- the value of the slope of the measurement curve is checked and a breaking point corresponding to a sharp increase of said slope value is determined,
- the flow rate corresponding to the breaking point is determined.

8. Method according to claim 7, **characterized in that** the device comprising a first flow meter (15) arranged between the injection hole (8) and the collecting hole (10) to measure the speed of transit of the liquid in the soil along the first hollow tube, said speed is measured at least at the breaking point.

## Patentansprüche

1. Vorrichtung zur Bodenerosionsmessung auf einer unter der Oberfläche gelegenen Bodenhöhe, **dadurch gekennzeichnet, dass** sie umfasst:
- Mittel zum Einspritzen (7) einer Flüssigkeit, die mit einem ersten Ende eines in den Boden geführten ersten Einspritz-Hohlrohrs (2) verbunden sind, wobei das genannte erste Hohlrohr an einem zweiten Ende eine Spitze (6) umfasst, über der mindestens eine Öffnung zum Einspritzen (8) der Flüssigkeit in den Boden vorgesehen ist, welche Einspritzmittel Überwachungs- und Messvorrichtungen für den Einspritzdurchsatz aufweisen,
- Mittel zum Auffangen der Flüssigkeit aus dem Boden auf der genannten unteren Höhe, die zum einen mindestens eine Auffangöffnung (10) für die Flüssigkeit umfassen, die mit einer Kammer (9) verbunden ist, und andererseits Fördermittel zu einem an der Oberfläche vorgesehenen Trübungsmesser (14) für die in der Kammer aufgefangene Flüssigkeit, wobei die Fördermittel ein zweites Hohlrohr (12), zum Ausstoßen, umfassen, dessen Achse im Wesentlichen parallel zum ersten Einspritz-Hohlrohr (2) ist.

2. Vorrichtung nach Anspruch 1, **dadurch gekennzeichnet, dass** das erste Einspritz-Hohlrohr (2) und das zweite Ausstoß-Hohlrohr (12) koaxial zueinander sind, das zweite Ausstoß-Hohlrohr (12) innerhalb des ersten Einspritz-Hohlrohrs (2) angeordnet ist, die Auffangöffnung (10) zwischen der Spitze (6) des ersten Rohrs (2) und der Einspritzöffnung (8) für die Flüssigkeit vorgesehen ist, wobei sich die Kammer (9) in der Spitze (6) befindet, sowie **dadurch**, dass ein erster Durchflussmesser (15) zwischen der Einspritzöffnung (8) und der Auffangöffnung (10) angeordnet ist, um die Durchflussgeschwindigkeit der Flüssigkeit im Boden entlang dem ersten Hohlrohr zu messen.

3. Vorrichtung nach Anspruch 1 bis 2, **dadurch gekennzeichnet, dass** es einen Aufnahmebehälter (13) zwischen dem zweiten Ausstoß-Hohlrohr (12) und Mitteln zum Befüllen des Trübungsmessers (14) umfasst.

4. Vorrichtung nach Anspruch 1 bis 3, **dadurch gekennzeichnet, dass** die Fördermittel für die Flüssigkeit eine Förderpumpe (16) mit einem Eintritt (16a) umfassen, der mit der Kammer und einem Auslass (16b) verbunden ist, der mit dem zweiten Ausstoß-Hohlrohr (12) verbunden ist.

5. Vorrichtung nach einem der Ansprüche 1 bis 3, **dadurch gekennzeichnet, dass** die Fördermittel für die Flüssigkeit einen Luftverdichter (17) umfassen, der mit einem zum zweiten Ausstoß-Hohlrohr (12) koaxialen Innenrohr (18) verbunden ist, das in die Kammer mündet, um die Flüssigkeit durch aufsteigende Luft aufzufangen.

6. Vorrichtung nach Anspruch 5, **dadurch gekennzeichnet, dass** das zweite Hohlrohr (12) einen zweiten Durchflussmesser mit Thermoresistenz umfasst.

7. Verfahren zur Herstellung einer Vorrichtung nach einem der Ansprüche 1 bis 6, **dadurch gekennzeichnet, dass**
- das erste Einspritz-Hohlrohr (2) und die Aufnahmemittel in den Boden (3) bis zu einer Höhe unter der Oberfläche eingeführt werden,
- die Flüssigkeit in regelmäßigen Abständen entsprechend einem gegebenen Durchsatz in das erste Einspritz-Hohlrohr (2) eingespritzt wird, und zwar mit Hilfe der Einspritzmittel (7), indem der Durchsatz entsprechend einem vorbestimmten Erhöhungswert zwischen zwei Einspritzungen erhöht wird,
- bei jeder Einspritzung eine Trübungsmessung des mittels der Auffangmittel aufgefangenen Flüssigkeit vorgenommen wird,
- eine Messkurve erstellt wird, die den Durchsatz den gemessenen Trübungswerten zuordnet,
- der Wert der Neigung der Messkurve überwacht und ein Schwellenwert bestimmt wird, der einem plötzlichen Anstieg des genannten Neigungswerts entspricht,
- der dem Schwellenwert entsprechende Durchsatz bestimmt wird.

8. Verfahren nach Anspruch 7, **dadurch gekennzeichnet, dass**, nachdem die Vorrichtung einen ersten Durchflussmesser (15) umfasst, der zwischen der Einspritzöffnung (8) und der Auffangöffnung (10) zum Messen der Bewegungsgeschwindigkeit der Flüssigkeit im Boden entlang dem ersten Hohlrohr angeordnet ist, die Geschwindigkeit zumindest beim Schwellenwert gemessen wird.
